# EUROPÄISCHE PATENTSCHRIFT

(11) **EP 1 028 652 B1**
(45) Veröffentlichungstag und Bekanntmachung des Hinweises auf die Patenterteilung: **03.01.2007**
(21) Anmeldenummer: 98963323.5
(22) Anmeldetag: 05.11.1998
(51) Int. Cl.: A61B 5/0468, G06F 17/00

(54) **AUSWERTEN VON ELEKTROKARDIOGRAMMEN IM BEREICH VON EXTRASYSTOLEN**
EVALUATION OF ELECTROCARDIOGRAMS IN THE FIELD OF EXTRASYSTOLES
ANALYSE DES ELECTROCARDIOGRAMMES EN CE QUI CONCERNE LES EXTRASYSTOLES

(30) Priorität: 07.11.1997 DE 19749393
(43) Veröffentlichungstag der Anmeldung: 23.08.2000
(73) Patentinhaber: Schmidt, Georg, 80796 München (DE)
(72) Erfinder: Schmidt, Georg, 80796 München (DE)
(74) Vertreter: Lindner-Vogt, Karin L.
(86) Internationale Anmeldenummer: PCT/DE1998/003225
(87) Internationale Veröffentlichungsnummer: WO 1999/023944

(56) Entgegenhaltungen:
- DE-A- 2 458 475
- GB-A- 2 190 505
- US-A- 4 499 904

## Beschreibung

Die Erfindung bezieht sich auf ein Verfahren und eine Vorrichtung zum Auswerten von Elektrokardiogrammen (EKG) im Bereich von Extrasystolen.

Extrasystolen sind außerhalb des regulären Grundrhythmus vorzeitig auftretende Herzschläge; sie können einzeln, zu zweit als sogenannte Couplets oder gehäuft als Salven auftreten. Nach ihrem Entstehungsort werden die Extrasystolen im wesentlichen in Vorhof- oder supraventrikuläre Extrasystolen und Kammer- oder ventrikuläre Extrasystolen unterteilt. Ursache und Entstehung der Extrasystolen sind bis heute noch nicht vollständig verstanden. Es liegt jedoch ein komplexes Zusammenspiel lokaler Faktoren (im Herzmuskel) und übergeordneter Strukturen (im autonomen Nervensystem) vor.

Aus der US-A-4,499,904 ist eine Vorrichtung bekannt, mit der aus dem Elektrokardiogramm Extrasystolen bestimmt werden können. Hierzu werden die Zeitintervalle aufeinanderfolgender Herzschläge aufgetragen und mit einem Referenzwert verglichen; weicht das gerade bestimmte Zeitintervall deutlich von dem Referenzwert ab, so wird dieses Zeitintervall markiert und gegebenenfalls angezeigt. Wesentlicher Gedanke dieser Vorrichtung ist es, bei einer Langzeitüberwachung die sich ansammelnden Daten zu komprimieren und zu reduzieren, ohne dass der Aussageinhalt über derartige abnorme Herzschläge verloren geht. Mit dieser Vorrichtung werden somit im wesentlichen nur Extrasystolen erfasst; eine weitergehende Auswertung ist nicht vorgesehen.

Extrasystolen werden nach Art, nach der Häufigkeit ihres Auftretens, nach ihrer Form bzw. nach ihrem Entstehungsort klassifiziert. Bei der Abschätzung der Prognose von Personen, die gerade einen Herzinfarkt erlitten haben, hat der Nachweis häufiger und repetitiver Extrasystolen eine gewisse Bedeutung erlangt. Allerdings ist der Nachweis von Extrasystolen im Langzeit-EKG wenig spezifisch; Extrasystolen treten nicht nur bei kranken auf, sondern auch bei gesunden Menschen. Daher ist es schwierig, wenn nicht unmöglich, beim einzelnen Patienten von einer Extrasystolie therapeutische Konsequenzen abzuleiten, z.B. die prophylaktische Implantation eines Cardioverter-Defibrillators. Aus diesem Grunde wurden bei der Auswertung von Elektrokardiogrammen die auftretenden Extrasystolen ggf. mit den benachbarten Herzschlägen aus dem Elektrokardiogramm ausgeblendet wurden, da die Extrasystolen als Artefakte betrachtet wurden, und nur der Grundrhythmus der Herzschläge betrachtet wurde. Eine zuverlässige Risikostratifizierung ist hiermit nicht möglich.

Auch die übrigen verfügbaren Verfahren zur Risikostratifizierung sind relativ unzuverlässig.

Es wäre ein großer Fortschritt, wenn man das individuelle Risiko für eine Person durch Parameter, die mit Extrasystolen gekoppelt sind, genauer als bisher bestimmen könnte.

Es ist daher Aufgabe der Erfindung, ein Verfahren und eine Vorrichtung zur Auswertung von Elektrokardiogrammen im Bereich von Extrasystolen anzugeben, um Parameter zur Verfügung zu stellen, die dem individuellen Risiko einer Person zugeordnet werden können.

Diese Aufgabe ist gemäß der Erfindung für ein Verfahren bzw. eine Vorrichtung der in Rede stehenden Art durch die Merkmale der unabhängigen Patentansprüche gelöst.

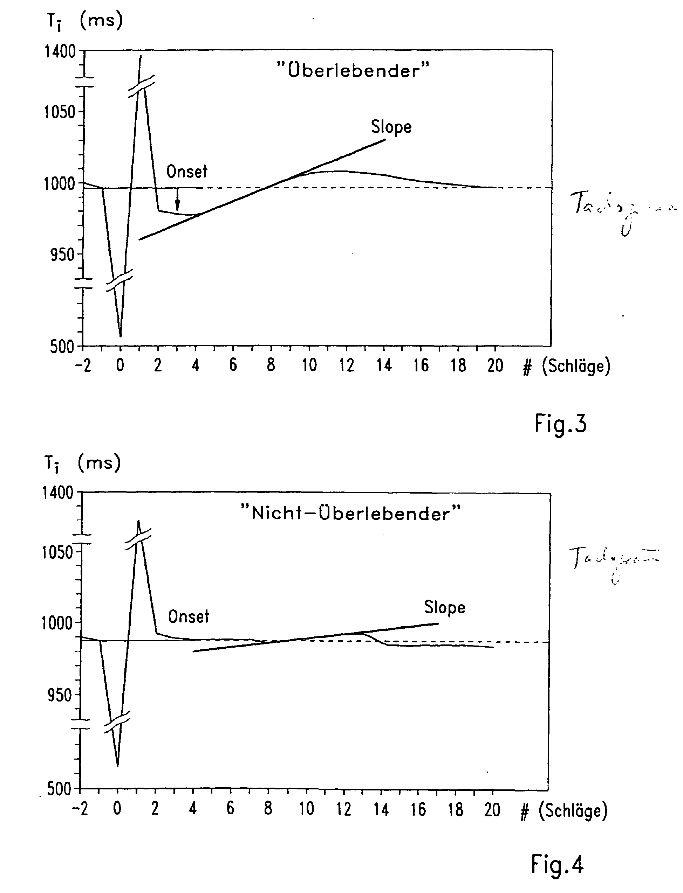

Im Gegensatz zu herkömmlichen Auswerteverfahren, bei denen die Extrasystolen als "störende" Artefakte betrachtet wurden, hat es sich gezeigt, dass Extrasystolen charakteristische Signaturen im Grundrhythmus der Herzschläge hinterlassen, die sich zur Risikostratifizierung nutzen lassen. Bei Personen mit normalem oder nur gering erhöhtem Risiko kommt es in der Regel unmittelbar nach einer Extrasystole zu einer Beschleunigung der Herzschlagfolge, die allerdings nur für einige Herzschläge anhält (transiente Frequenzzunahme) und die dann von einer Phase der Frequenzabnahme der Herzschlagfolge gefolgt wird. Nach etwa zehn bis zwanzig Herzaktionen pendelt sich die Herzfrequenz wieder in ihrem Ausgangsniveau ein (transiente Frequenzabnahme, siehe Fig. 3). Bei Personen mit erhöhtem Risiko ist diese charakteristische Reaktion deutlich abgeschwächt oder gänzlich aufgehoben. In diesen Fällen findet sich häufig eine mehr oder weniger erratische, d. h. ungeordnete Herzschlagfolge. Eine Auswertung eines EKG gerade in der bisher nicht berücksichtigten Umgebung von Extrasystolen führt zu weit zuverlässigeren Aussagen als dieses bisher möglich war.

Die Quantifizierung dieser Unterschiede ist grundsätzlich auf verschiedene Arten möglich, beispielsweise mit Analyseverfahren in der Zeitdomäne oder in der Frequenzdomäne.

In der Zeitdomäne haben sich die folgenden Parameter als zur Risikoabschätzung geeignet erwiesen:
"Onset": Differenz der Mittelwerte der letzten normalen RR-Intervalle vor der Extrasystole und der ersten normalen RR-Intervalle nach der Extrasystole; vorzugsweise werden hierbei jeweils zwei RR-Intervalle unmittelbar vor bzw. nach der Extrasystole verwendet.
"Slope": größte Frequenzverlangsamung innerhalb einer Sequenz von mehreren, z. B. 20 Herzschlagintervallen nach einer Extrasystole über vorzugsweise fünf sukzessive RR-Intervalle ermittelt als Steigung der Regressionsgerade.
"Korrelationskoeffizient des Slope": Maß für die Regelmäßigkeit des Slope und gebildet durch numerische Mittelwertbildung mehrerer aufeinanderfolgender Slope-Werte.

Alle genannten Prameter haben sich zur Risikostratifizierung als geeignet erwiesen: Bei geringem Onset, flachem Slope oder niedrigem Korrelationskoeffizienten des Slope ist das Risiko, im weiteren Verlauf zu versterben, signifikant erhöht. Bei deutlichem Onset, steilem Slope oder hohem Korrelationskoeffizienten des Slope ist das Risiko normal bzw. signifikant niedriger.

In der Frequenzdomäne können die niedrig- und hochfrequenten Anteile quantifiziert und ihr Verhältnis bestimmt werden: Bei Zunahme der hochfrequenten Anteile ist das Risiko, im weiteren Verlauf zu versterben, signifikant erhöht. Bei Zunahme der niederfrequenten Anteile ist das Risiko normal bzw. signifikant niedriger.

Die neuen Risikoparameter erwiesen sich bei multivarianter Analyse als weitgehend unabhängig von allen anderen bisher verwendeten Risikoparametern. Dies bedeutet, dass ein wesentlicher Anteil der in ihnen enthaltenen Information tatsächlich neu, d.h. klinisch additiv ist.

Bei der Untersuchung von Personen mit Vorhofextrasystolen konnten derartige Assoziationen von Slope und Korrelationskoeffizienten des Slope mit dem Mortalitätsrisiko ebenfalls festgestellt werden.

Eine Vorrichtung zum Auswerten von Elektrokardiogrammen im Bereich von Extrasystolen kann im übrigen direkt in ein Gerät zum Aufzeichnen von Elektrokardiogrammen, insbesondere Langzeitelektrokardiogrammen integriert werden. Dieses Gerät liefert dann nicht nur das eigentliche EKG, sondern bereits die Auswertung hinsichtlich des Zeitverhaltens der Herzschläge vor und insbesondere nach einer Extrasystole. Auch die Integration einer Vorrichtung gemäß der Erfindung in einen Herzschrittmacher oder in einen implantierten Defibrillator ist denkbar.

Extrasystolen sind im EKG in der Regel leicht identifizierbar, da sich das Zeitverhalten gegenüber dem regelmäßigen Grundrhythmus und - im Falle von Kammerextrasystolen - ihre Form deutlich sichtbar ändert. Daher kann eine Folge vor einer Extrasystole eindeutig von einer Folge nach einer Extrasystole unterschieden werden.

Es ist im übrigen auch möglich, das Verfahren gemäß der Erfindung für klinische Untersuchungen hinsichtlich der Wirkung von Medikamenten heranzuziehen. Es zeigte sich, dass sich mit Hilfe von Onset, Slope und Korrelationskoeffizienten des Slope Patienten mit unterschiedlicher Wirksamkeit eines antiarrhythmischen Medikamentes identifizieren lassen. Je deutlicher bei der Behandlung von Patienten Onset, Slope und Korrelationskoeffizient ausgeprägt sind, desto wirksamer wird das Medikament eingestuft.

Weitere Ausgestaltungen der Erfindung gehen aus den Unteransprüchen hervor.

Die Erfindung ist anhand der Zeichnung näher erläutert. In dieser stellen dar:
Figur 1 eine Vorrichtung zum Auswerten von Elektrokardiogrammen im Bereich von Extrasystolen gemäß der Erfindung;
Figur 2 einen Ausschnitt aus einem 24-Stunden-Elektrokardiogramm eines Postinfarktpatienten im Bereich einer ventrikulären Extrasystole;
Figur 3 ein Diagramm zur Darstellung der Zeitintervalle zwischen jeweils zwei aufeinanderfolgenden Herzschlägen im Bereich um eine Extrasystole bei einem Patienten mit niedrigem Mortalitätsrisiko;
Figur 4 ein Diagramm zur Darstellung der Zeitintervalle zwischen jeweils zwei aufeinanderfolgenden Herzschlägen im Bereich um eine Extrasystole bei einem Patienten mit hohem Mortalitätsrisiko;
Figur 5 ein Diagramm der Überlebenswahrscheinlichkeit von Patienten mit deutlichem und abgeschwächtem bzw. aufgehobenen Onset.
Figur 6 ein Diagramm der Überlebenswahrscheinlichkeit von Patienten mit deutlichem und abgeschwächtem bzw. aufgehobenen Slope.

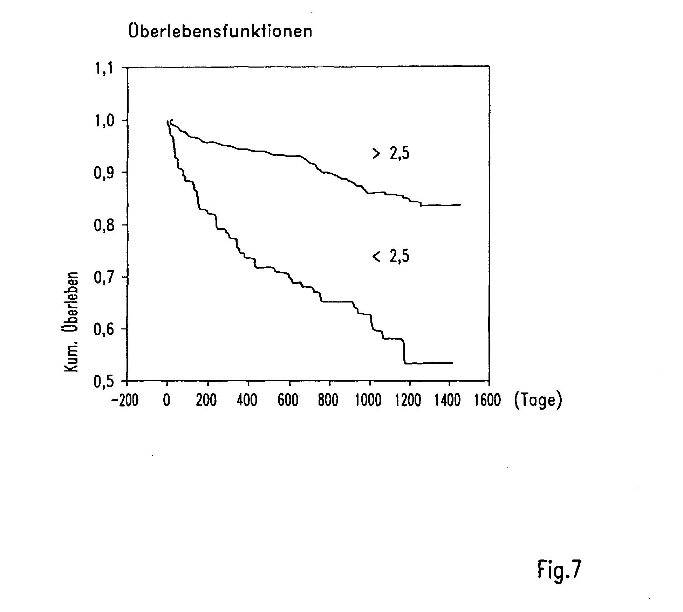

Figur 7 ein Diagramm der Überlebenswahrscheinlichkeit von Patienten mit hohem und niedrigem Korrelationskoeffizienten des Slope.

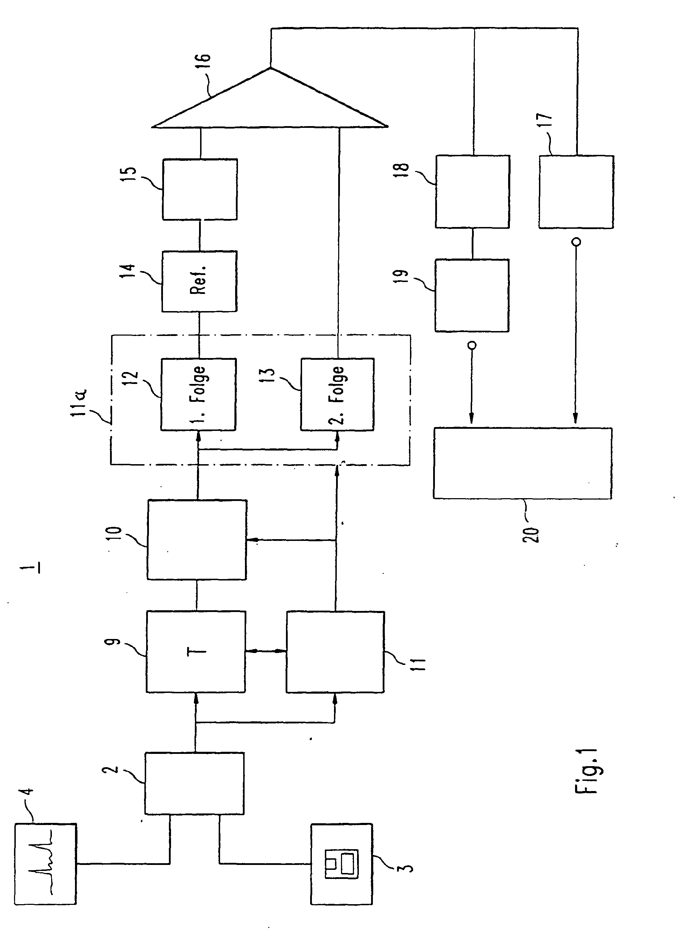

In Figur 1 ist eine Vorrichtung 1 zum Auswerten von Elektrokardiogrammen dargestellt. Diese Vorrichtung hat eine Eingabeeinheit 2, der Daten eines Langzeitelektrokardiogrammes zugeführt werden. Dieses erfolgt entweder über einen Datenträger 3, auf dem ein oder mehrere Langzeitelektrokardiogramme aufgezeichnet sind, oder über einen Elektrokardiographen 4, mit dem ein Langzeitelektrokardiogramm einer Person aufgezeichnet wird.

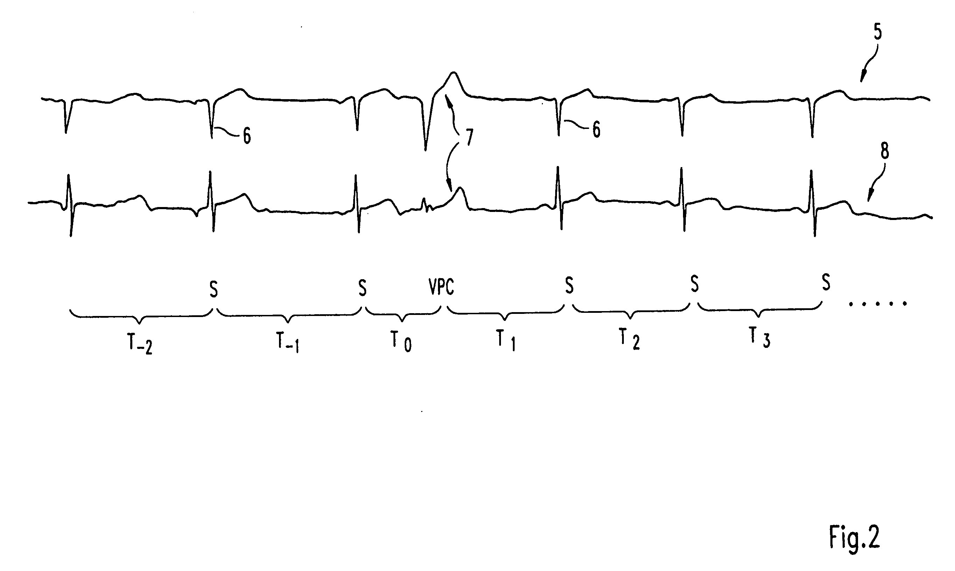

In Figur 2 ist in der oberen Zeile ein Ausschnitt aus einem EKG 5 gezeigt, aus dem der Grundrhythmus durch die R-Zacken 6 erkennbar ist. Zwischen der zweiten und dritten Zacke ist eine ventrikuläre Extrasystole (VES) 7 sichtbar, durch die der reguläre Grundrhythmus der Herzschläge gestört wird. In der zweiten Zeile der Figur 2 ist eine Ableitung des EKG 5 dargestellt, aus der ebenfalls die markante Unterbrechung des regulären Grundrhythmus durch die Extrasystole 7 erkennbar ist.

In der dritten Zeile der Figur 2 sind die Zeitintervalle zwischen den einzelnen Herzschlägen aufgezeigt, nämlich die Zeitintervalle T-2 und T-1 unmittelbar vor der Extrasystole, dann das verkürzte Zeitintervall T0 zwischen dem letzten Herzschlag vor der Extrasystole und der Extrasystole und dann anschließend die Zeitintervalle T1 zwischen Extrasystole und erstem regulären Herzschlag sowie anschließend zwischen jeweils zwei wiederum relativ regulären Herzschlägen T2, T3, ...

Diese Intervalle Ti werden in der Vorrichtung 1 durch eine Intervallmessschaltung 9 bestimmt; die Folge der Intervalle wird einem ersten Speicher 10 zugeführt. In einer Diskriminatorschaltung 11, der ebenfalls die Daten des EKG zugeführt werden und die in Verbindung mit der Messschaltung 9 steht, wird anhand der Zeitvorgaben des EKG und der zeitlichen Ableitung entsprechend dem Kurvenzug 8 die Extrasystole erkannt und markiert. Dieses Ergebnis wird dem ersten Speicher 10 und einer Auftrennschaltung 11a zugeführt, wo die Folge der Herzschläge in eine erste Folge unmittelbar vor der Extrasystole und in eine zweite Folge unmittelbar nach der Extrasystole aufgetrennt wird. Diese beiden Folgen werden in jeweils einem Speicher 12 bzw. 13 abgelegt. Aus einer bestimmten Anzahl von Zeitintervallen aus der ersten Folge, in diesem Falle der Zeitintervalle T-2 und T-1 unmittelbar vor der Extrasystole wird in einem Mittelwertbildner 14 ein Mittelwert aus diesen Zeitintervallen gebildet und als Referenzwert R in einem Speicher 15 abgelegt.

Die Zeitintervalle unmittelbar nach der Extrasystole, also die Zeitintervalle T1, T2, T3 usw. werden in einer Vergleichsschaltung 16 mit dem im Speicher 15 vorliegenden Mittelwert verglichen. Am Ausgang der Vergleichsschaltung wird jeweils die Abweichung bzw. Differenz zwischen beiden Werten und die Nummer des jeweiligen Zeitintervalls aus der Folge und der Extrasystole ausgegeben, einer ersten Ausgabeeinheit 17 zugeführt und dort gegebenenfalls angezeigt. Eine schematische Anzeige ist in den Figuren 3 und 4 dargestellt: Längs der Ordinate dieser Diagramme ist die Zeit in Millisekunden für die jeweils gemessenen Zeitintervalle aufgetragen, längs der Abszisse sind die Nummern, d.h. die Indizes der einzelnen Zeitintervalle äquidistant aufgetragen. Aus Figur 3 sieht man, dass nach den markanten Zeitabweichungen der Extrasystole 7 die Zeitintervalle T2, T3, T4 und so fort zwischen wieder annähernd regelmäßig auftretenden R-Zacken zunächst verkürzt werden. Anschließend bildet sich etwa ab dem Zeitintervall T5 eine Gegenreaktion aus, wobei die Zeitintervalle ab dem Zeitintervall T7 länger als der Referenzwert werden. Anschließend schwingt das Zeitverhalten in Richtung auf den Referenzwert, der in diesem Falle etwa ab dem Zeitintervall T18 erreicht wird. Die einzelnen Messpunkte können durch einen annähernd glatten Kurvenzug miteinander verbunden werden.

Das in Figur 4 gezeigte Diagramm stammt von einem anderen Patienten: Hier wird deutlich, dass sich die Zeitintervalle nach der Extrasystole 7 quasi nicht von dem Mittelwert entfernen; es sind nur geringfügige, annähernd statistische, Abweichungen vorhanden.

Die in der Vergleichsschaltung 16 ermittelten Daten werden zusätzlich einer Graphenauswerteschaltung 18 zugeführt, wobei dieses natürlich auch über die Ausgabeeinheit 17 erfolgen kann. In dieser Auswerteschaltung 18 wird der durch eine Verbindung aller Messpunkte der in den Figuren 3 und 4 erzeugte Graph hinsichtlich seiner Steigung gegenüber der Abszisse ausgewertet. Dies erfolgt nach einem einfachen Algorithmus mit einigen Vorgaben, z.B. in diesem Falle die Auswertung jeweils von fünf aufeinanderfolgenden Messpunkten. In dieser Graphenauswertung 18 wird dann derjenige Abschnitt des Graphen markiert, der gegenüber der Abszisse die größte Steigung, d.h. den größten Slope aufweist. Auch dieses ist in den Figuren 3 und 4 eingetragen. Man sieht, dass bei dem Graphen gemäß Figur 3 der Abschnitt zwischen den Zeitintervallen T5 und T9 eine markante Steigung gegenüber der Abszisse annimmt, wohingegen eine solche Steigung bei dem Graphen gemäß Figur 4 praktisch nicht vorhanden ist.

Die geschilderte Graphenauswertung kann anhand eines einzigen Graphen nach einer Extrasystole erfolgen; signifikantere Aussagen werden jedoch erzielt, wenn mehrere Graphen (im Falle mehrerer Extrasystolen) übereinander gelegt werden und nach einer gewissen Normierung hinsichtlich des Referenzwertes gemittelt werden. Die Tendenz, ob eine signifikante Steigung auftritt oder nicht, wird durch eine solche Superposition wesentlich deutlicher. Das Ergebnis der Graphenauswertung 18 wird auf eine zweite Ausgabeeinheit 19 geleitet und kann in herkömmlicher Weise ausgegeben, z.B. angezeigt oder ausgedruckt werden. Die in den Ausgabeeinheiten 17 und 19 vorliegenden Ergebnisse können des weiteren z.B. auf einem Datenträger 20 abgespeichert werden.

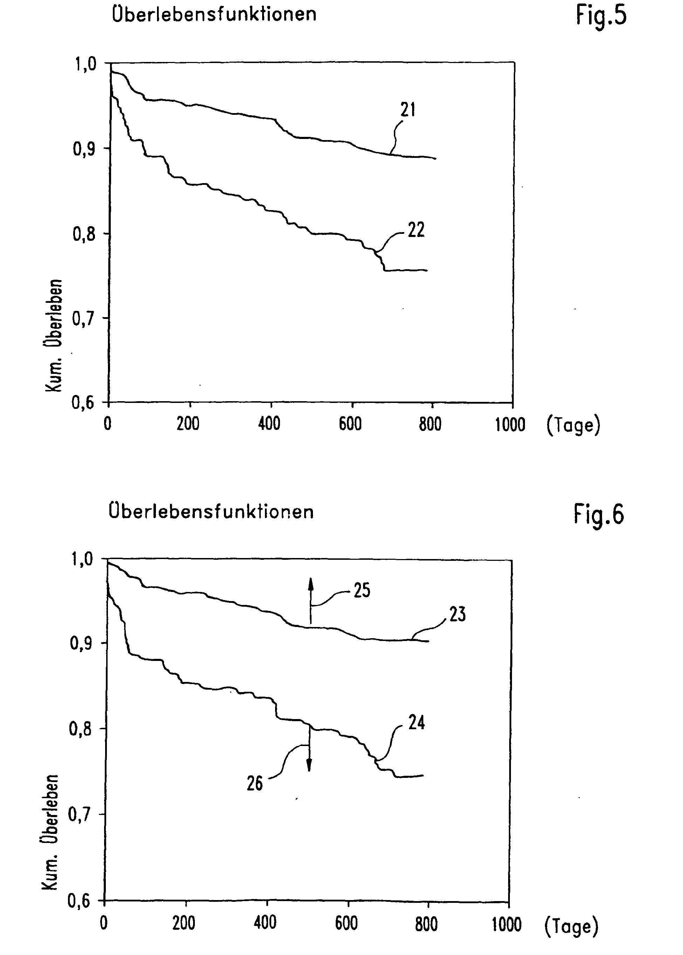

In den Figuren 5 und 6 ist aus den Daten von mehreren tausend EKG-Langzeituntersuchungen an Postinfarktpatienten die Überlebensfunktion dieser Patienten über der Zeit in Tagen aufgetragen. In der Figur 5 ist der Kurvenzug 21 denjenigen Patienten zugeordnet, bei denen das Zeitverhalten der Herzschläge unmittelbar nach einer Extrasystole hinsichtlich des Onset signifikant von dem Referenzwert abwich, während der Kurvenzug 22 die Überlebensfunktion von Patienten darstellt, bei denen keine signifikante Abweichung auftrat, d.h. dass Ergebnisse entsprechend den Figuren 3 bzw. 4 vorlagen. Man sieht, dass von den Patienten, bei denen eine signifikante Abweichung vorlag, nach einem Jahr etwa noch 95% lebten, während dies nur für 85% der Patienten gilt, bei denen keine signifikante Abweichung gemessen wurde.

In Figur 6 sind zwei ähnliche Kurvenzüge 23 und 24 gezeigt, bei denen die Überlebensfunktion unter Berücksichtigung der oben geschilderten Steigung bzw. Slope aufgetragen ist. Auch hier sieht man, dass nach etwa einem Jahr bei Patienten mit signifikantem Slope noch etwa 95% lebten, während bei Patienten mit keiner oder geringer Slope dieser Anteil auf 85% abgefallen ist. Die Kurven der Figuren 5 und 6 zeigen vergleichbare Ergebnisse, offensichtlich liefern für den Fall der Kammerextrasystolen beide Parameter gute Aussagen.

Dies bedeutet für die Auswertung von Elektrokardiogrammen einer Person, dass bei deutlichem Onset und Slope nach einer Extrasystole auf ein relativ geringes Mortalitätsrisiko geschlossen werden kann, wohingegen bei nur geringen oder gar keinen Abweichungen im Onset und einem nicht signifikanten Slope auf ein relativ hohes Mortalitätsrisiko geschlossen werden kann. Entsprechend dieser Auswertung kann dann ein Arzt gegebenenfalls in Verbindung mit anderen Untersuchungen eine Diagnose erstellen und eine geeignete Therapie der untersuchten Person einleiten.

Das angegebene Auswerteverfahren für Onset und Slope kann unmittelbar für die klinische Erprobung von Medikamenten eingesetzt werden. Hierzu werden die Personengruppen in jeweils zwei Gruppen aufgeteilt, die signifikante Änderungen in Onset und Slope bzw. keine solch signifikanten Änderungen zeigen. Durch die Gabe eines Medikamentes kann nach einer Langzeituntersuchung anhand der vorliegenden statistischen Daten unmittelbar geschlossen werden, ob sich etwa eine Verbesserung der Überlebensfunktion einstellt, wie dieses etwa durch den Pfeil 25 in Figur 6 für die Patienten mit relativ stabiler physiologischer Kondition angedeutet ist, oder ob sich sogar eine Verschlechterung einstellt, wie dieses durch den nach unten gerichteten Pfeil 26 für Patienten ohne signifikante Änderung von Onset und Slope angedeutet ist.

Aufgrund der Auswertung von Tausenden von auf Datenträgern gespeicherten Elektrokardiogrammen scheint das Kriterium einer signifikanten Steigung oder Slope bei allen Extrasystolen ein deutliches Kriterium zu sein, während ein signifikanter Onset im wesentlichen nur bei Kammerextrasystolen ein ebenso deutliches Kriterium bildet. Bei Vorhofextrasystolen, d.h. im wesentlichen vorzeitigen normalen Herzschlägen, verändert sich zunächst nach der Extrasystole der Onset praktisch nicht; erst nach einigen Herzschlägen tritt eine Änderung auf, die sich in einer deutlichen Änderung der Steigung bzw. Slope zeigt.

In dem Vorhergehenden ist beschrieben worden, dass nach Erkennen einer Extrasystole die Zeitintervalle zwischen Herzschlägen vor und nach der Systole ausgewertet werden. Für die Parameter "Slope" und "Korrelationskoeffizient des Slope" werden die Zeitintervalle zwischen aufeinander folgenden Herzschlägen nach der Extrasystole ausgewertet, für den Parameter "Onset" werden die Zeitintervalle zwischen Herzschlägen vor der Extrasystole mit Zeitintervallen zwischen Herzschlägen nach der Extrasystole verglichen.

Wie ausgeführt, können auch andere Parameter für andere charakteristische Eigenschaften zur Auswertung eines Elektrokardiogrammes herangezogen werden, so z. B. das erwähnte Frequenzverhalten der einzelnen Herzschläge.

## Patentansprüche

1. Verfahren zum Auswerten von Elektrokardiogrammen von Patienten im Bereich von Extrasystolen, **dadurch gekennzeichnet,**
**dass** unmittelbar nach einer Extrasystole die Änderung der Länge der Zeitintervalle zwischen aufeinander folgenden Herzschlägen über eine definierte Anzahl von Herzschlägen bestimmt und das Ergebnis für eine Risikostratifizierung für einen Patienten herangezogen wird.

2. Verfahren nach Anspruch 1, **dadurch gekennzeichnet, dass**
für die Änderung der Länge der Zeitintervalle zwischen aufeinander folgenden Herzschlägen als ein erster Parameter die Frequenzverlangsamung innerhalb einer Folge von mehreren Herzschlägen nach einer Extrasystole bestimmt wird ("slope") und/oder
als ein zweiter Parameter durch Mittelwertbildung mehrerer aufeinander folgender Messwertfolgen jeweils nach unterschiedlichen Extrasystolen ein die Regelmäßigkeit des slope anzeigender Korrelationskoeffizient bestimmt wird und/oder
als ein dritter Parameter die Differenz zwischen einem Referenzwert entsprechend zumindest einem Zeitintervall oder entsprechend einem aus mehreren Zeitintervallen gemittelten Wert aus der Folge vor der Extrasystole und dem gegebenenfalls gemittelten Zeitintervall zwischen den ersten Herzschlägen unmittelbar nach der Extrasystole gebildet wird ("onset")
und dass zumindest einer dieser Parameter zur Risikostratifizierung für den Patienten quantifiziert wird.

3. Verfahren nach Anspruch 1 oder 2, **dadurch gekennzeichnet, dass** die Messergebnisse für die Folge von Zeitintervallen nach unterschiedlichen Extrasystolen gegebenenfalls nach Normierung superpositioniert und die Messwertfolgen gemittelt werden.

4. Verfahren nach Anspruch 2 oder 3, **dadurch gekennzeichnet, dass** die Parameter "slope", Korrelationskoeffizient und "onset" aufgrund einer Mittelung nach Anspruch 3 bestimmt werden.

5. Verfahren nach einem der vorhergehenden Ansprüche, dass eine auszuwertende Folge von Zeitintervallen unmittelbar vor der Extrasystole ein bis fünf, vorzugsweise zwei Zeitintervalle und/oder eine Folge unmittelbar nach der Extrasystole bis 50, vorzugsweise zwischen 10 und 20 Zeitintervalle umfasst.

6. Verfahren nach einem der vorhergehenden Ansprüche, **dadurch gekennzeichnet, dass** die Auswertung der Änderung der Länge der Zeitintervalle zwischen einzelnen Herzschlägen in der Zeitdomäne und/oder in der Frequenzdomäne erfolgt.

7. Verfahren nach Anspruch 6, **dadurch gekennzeichnet, dass** in Langzeituntersuchungen das Zeit- und/oder Frequenzverhalten in einer Folge von aufeinander folgenden Herzschlägen vor und nach Extrasystolen für eine Vielzahl von Patienten untersucht wird, und dass die Ergebnisse in Gruppen unterteilt werden, die für jeweils mehrere Patienten ein ähnliches Zeit- und/oder Frequenzverhalten zeigen.

8. Vorrichtung zum Auswerten von Elektrokardiogrammen im Bereich von Extrasystolen zur Durchführung des Verfahrens nach Anspruch 1, **dadurch gekennzeichnet, dass** eine Einrichtung (9 bis 17) zum Erfassen der Änderung der Länge der Zeitintervalle zwischen aufeinander folgenden Herzschlägen über eine definierte Anzahl von Herzschlägen unmittelbar nach einer Extrasystole vorgesehen ist und eine Einrichtung (18 bis 20) zum Auswerten und Quantifizieren dieser Ergebnisse für eine Risikostratifizierung für einen Patienten vorgesehen ist.

9. Vorrichtung nach Anspruch 8, **dadurch gekennzeichnet, dass** eine Einrichtung (11, 12, 16 bis 20) zum Vergleich der Zeitintervalle zwischen Herzschlägen nach der Extrasystole mit zumindest einem Zeitintervall zwischen zwei Herzschlägen vor der Extrasystole beziehungsweise einem Mittelwert aus mehreren Zeitintervallen vor der Extrasystole vorgesehen ist.

10. Vorrichtung nach Anspruch 8 oder 9, **gekennzeichnet durch** folgende Merkmale:
eine erste Einrichtung (9) zum Bestimmen der Zeitintervalle (Tᵢ) zwischen zwei jeweils aufeinander folgenden Herzschlägen (6, 7) einer zusammen hängenden Folge von Herzschlägen;
einen ersten Speicher (10) zum Speichern der Zeitintervalle dieser Folge;
eine zweite Einrichtung (11) zum Markieren einer Extrasystole (7);
eine dritte Einrichtung (11a) zum Auftrennen der gespeicherten Zeitintervalle bei Auftreten einer Extrasystole in jeweils eine Folge unmittelbar vor und eine unmittelbar nach der Extrasystole;
eine vierte Einrichtung (14) zum Bilden eines Referenzwertes aus zumindest einem Zeitintervall aus der Folge vor der Extrasystole;
einen zweiten Speicher (15) zum Speichern dieses Referenzwertes;
eine fünfte Einrichtung (16) zum Vergleichen der Zeitintervalle aus der Folge unmittelbar nach der Extrasystole mit dem gespeicherten Referenzwert;
eine sechste Einrichtung (17) zum Ausgeben der Vergleichsergebnisse.

11. Vorrichtung nach Anspruch 10, **dadurch gekennzeichnet, dass** die dritte Einrichtung (11a) zwei Speicher (12, 13) zum Speichern der ersten bzw. zweiten Folge von Zeitintervallen aufweist.

12. Vorrichtung nach Anspruch 10, **dadurch gekennzeichnet, dass** die vierte Einrichtung (14) aus zumindest zwei aufeinander folgenden Zeitintervallen unmittelbar vor der Extrasystole als Referenzwert deren Mittelwert bildet.

13. Vorrichtung nach Anspruch 10, **dadurch gekennzeichnet, dass** der Ausgang der fünften Vergleichseinrichtung (15, 16) mit einer Graphenauswerteschaltung (18) zum Berechnen des tendenziellen Verlaufs einer Folge aus einer bestimmten Anzahl von aufeinander folgenden Zeitintervallen nach einer Extrasystole gegenüber dem Referenzwert verbunden ist.

## Claims

1. A method for analyzing electrocardiograms of patients in the area of extrasystoles, **characterized in that** directly after an extrasystole, the change of the length of the time intervals between sequential heartbeats is determined over a defined number of heartbeats and the result is used for a risk stratification for a patient.

2. The method according to Claim 1, **characterized in that**, for the change of the length of the time intervals between sequential heartbeats, the frequency deceleration within a sequence of multiple heartbeats after an extrasystole ("slope") is determined as a first parameter, and/or a correlation coefficient indicating the regularity of the slope is determined as a second parameter by averaging multiple sequential measured value sequences, each after different extrasystoles, and/or the difference between a reference value corresponding to at least one time interval or corresponding to a value averaged from multiple time intervals is calculated from the sequence before the extrasystole and the possibly averaged time interval between the first heartbeats directly after the extrasystole as a third parameter ("onset"), and at least one of these parameters is quantified for risk stratification for the patient.

3. The method according to Claim 1 or 2, **characterized in that** the measurement results for the sequence of time intervals after different extrasystoles are superimposed, possibly after standardization, and the measured value sequences are averaged.

4. The method according to Claim 2 or 3, **characterized in that** the parameters "slope", correlation coefficient, and "onset" are determined on the basis of averaging according to Claim 3.

5. The method according to one of the preceding claims, **characterized in that** a sequence of time intervals to be analyzed directly before the extrasystole comprises one to five, preferably two time intervals, and/or a sequence of time intervals to be analyzed directly after the extrasystole comprises up to 50, preferably between 10 and 20 time intervals.

6. The method according to one of the preceding claims, **characterized in that** the analysis of the change the length of the time intervals between individual heartbeats is performed in the time domain and/or in the frequency domain.

7. The method according to Claim 6, **characterized in that**, in long-term examinations, the time and/or frequency behavior in a sequence of sequential heartbeats before and after extrasystoles is examined for multiple patients, and the results are subdivided into groups, each of which display similar time and/or frequency behavior for multiple patients.

8. A device for analyzing electrocardiograms in the area of extrasystoles for performing the method according to Claim 1, **characterized in that** an apparatus (9 through 17) is provided for detecting the change of the length of the time intervals between sequential heartbeats over a defined number of heartbeats directly after an extrasystole and an apparatus (18 through 20) is provided for analyzing and quantifying these results for a risk stratification for a patient.

9. The device according to Claim 8, **characterized in that** an apparatus (11, 12, 16 through 20) is provided for comparing the time intervals between heartbeats after the extrasystole to at least one time interval between two heartbeats before the extrasystole and/or a mean value of multiple time intervals before the extrasystole.

10. The device according to Claim 8 or 9, **characterized by** the following features:
a first apparatus (9) for determining the time intervals (Ti) between each two sequential heartbeats (6, 7) of a coherent sequence of heartbeats;
a first memory (10) for storing the time intervals of this sequence;
a second apparatus (11) for marking an extrasystole (7);
a third apparatus (11a) for partitioning the stored time intervals upon occurrence of an extrasystole into a sequence directly before and a sequence directly after the extrasystole;
a fourth apparatus (14) for calculating a reference value from at least one time interval from the sequence before the extrasystole;
a second memory (15) for storing this reference value;
a fifth apparatus (16) for comparing the time intervals from the sequence strictly after the extrasystole to the stored reference value;
a sixth apparatus (17) for outputting the comparison results.

11. The device according to Claim 10, **characterized in that** the third apparatus (11a) has two memories (12, 13) for storing the first and second sequence of time intervals, respectively.

12. The device according to Claim 10, **characterized in that** the fourth apparatus (14) calculates the mean value of at least two sequential time intervals directly before the extrasystole as a reference value.

13. The device according to Claim 10, **characterized in that** the output of the fifth comparison apparatus (15, 16) is connected to a graph analysis circuit (18) for calculating the tendency curve of a sequence of a specific number of sequential time intervals after an extrasystole in relation to the reference value.

## Revendications

1. Procédé pour l'analyse d'électrocardiogrammes de patients dans la zone d'extrasystoles, **caractérisé en ce que**,
juste après une extrasystole, la variation de la longueur des intervalles de temps entre des battements cardiaques successifs est définie par un nombre défini de battements cardiaques et le résultat est utilisé pour une stratification de risque pour un patient.

2. Procédé selon la revendication 1, **caractérisé en ce que**
pour la modification de la longueur des intervalles de temps entre des battements cardiaques successifs, on détermine comme un premier paramètre le ralentissement de fréquence à l'intérieur d'une séquence de plusieurs battements cardiaques après une extrasystole ("slope") et/ou
en tant que second paramètre, on détermine un coefficient de corrélation indiquant la régularité du slope par la formation de valeur moyenne de plusieurs séquences de valeurs mesurées consécutives, à chaque fois après des extrasystoles différentes et/ou,
en tant que troisième paramètre, on fait la différence entre une valeur de référence correspondant à au moins un intervalle de temps ou correspondant à une valeur moyenne calculée à partir de plusieurs intervalles de temps à partir de la séquence avant l'extrasystole et l'intervalle de temps moyen éventuellement calculé entre les premiers battements cardiaques juste après l'extrasystole ("onset")
et **en ce qu'**on quantifie au moins l'un de ces paramètres pour la stratification de risque pour le patient.

3. Procédé selon la revendication 1 ou 2, **caractérisé en ce que** les résultats de la mesure sont superpositionnés pour la séquence d'intervalles de temps après différentes extrasystoles éventuellement après normalisation et les séquences de valeurs de mesure font l'objet d'un calcul de moyenne.

4. Procédé selon la revendication 2 ou 3, **caractérisé en ce que** les paramètres "slope", coefficient de corrélation et "onset" sont déterminés sur la base d'un calcul de moyenne selon la revendication 3.

5. Procédé selon les revendications précédentes, **caractérisé en ce qu'**une séquence à analyser d'intervalles de temps juste avant l'extrasystole comprend un à cinq, de préférence deux intervalles de temps et/ou une séquence juste après l'extrasystole jusqu'à 50, de préférence entre 10 et 20 intervalles de temps.

6. Procédé selon les revendications précédentes, **caractérisé en ce que** l'analyse de la longueur des intervalles de temps entre les battements cardiaques individuels s'effectue dans le domaine du temps et/ou dans le domaine de la fréquence.

7. Procédé selon la revendication 6, **caractérisé en ce que**, lors d'études de longue durée, on étudie le comportement en temps et/ou le comportement en fréquence dans une séquence de battements cardiaques consécutifs avant et après des extrasystoles pour une pluralité de patients et **en ce que** les résultats sont subdivisés en groupes, qui montrent un comportement en temps et/ou en fréquence semblable pour à chaque fois plusieurs patients.

8. Dispositif pour analyser des électrocardiogrammes dans le secteur d'extrasystoles pour mettre en oeuvre le procédé selon la revendication 1, **caractérisé en ce qu'**un dispositif (9 à 17) est prévu pour l'enregistrement de la modification de la longueur des intervalles de temps entre des battements cardiaques successifs sur un nombre défini de battements cardiaques juste après une extrasystole et un dispositif (18 à 20) est prévu pour l'analyse et la quantification de ces résultats pour une stratification de risque pour un patient.

9. Dispositif selon la revendication 8, **caractérisé en ce qu'**un dispositif (11, 12, 16 à 20) est prévu pour la comparaison des intervalles de temps entre des battements cardiaques après l'extrasystole avec au moins un intervalle de temps entre deux battements cardiaques avant l'extrasystole ou une valeur moyenne provenant de plusieurs intervalles de temps avant l'extrasystole.

10. Dispositif selon la revendication 8 ou 9, **caractérisé par** les caractéristiques suivantes :
un premier dispositif (9) pour déterminer les intervalles de temps (Tᵢ) entre deux battements cardiaques (6, 7) successifs d'une séquence cohérente de battements cardiaques ;
une première mémoire (10) pour la mémorisation des intervalles de temps de cette séquence ;
un second dispositif (11) pour le marquage d'une extrasystole (7) ;
un troisième dispositif (11a) pour la séparation des intervalles de temps mémorisés lors de l'apparition d'une extrasystole dans respectivement une séquence juste avant et une juste après l'extrasystole;
un quatrième dispositif (14) pour la formation d'une valeur de référence à partir d'au moins un intervalle de temps provenant de la séquence avant l'extrasystole ;
une seconde mémoire (15) pour la mémorisation de cette valeur de référence ;
un cinquième dispositif (16) pour la comparaison des intervalles de temps provenant de la séquence juste après l'extrasystole avec la valeur de référence mémorisée ;
un sixième dispositif (17) pour l'édition des résultats de la comparaison.

11. Dispositif selon la revendication 10, **caractérisé en ce que** le troisième dispositif (11a) présente deux mémoires (12, 13) pour le stockage de la première et de la seconde séquences d'intervalles de temps.

12. Dispositif selon la revendication 10, **caractérisé en ce que** le quatrième dispositif (14) forme à partir d'au moins deux intervalles de temps successifs juste avant l'extrasystole comme valeur de référence leur valeur moyenne.

13. Dispositif selon la revendication 10, **caractérisé en ce que** la sortie du cinquième dispositif de comparaison (15, 16) est reliée à un circuit d'analyse de graphe (18) pour le calcul de la courbe de tendance d'une séquence constituée d'un certain nombre d'intervalles de temps successifs après une extrasystole par rapport à la valeur de référence.
